# EUROPÄISCHE PATENTSCHRIFT

(11) **EP 2 037 871 B1**
(45) Veröffentlichungstag und Bekanntmachung des Hinweises auf die Patenterteilung: **24.02.2016**
(21) Anmeldenummer: 07786993.1
(22) Anmeldetag: 03.07.2007
(51) Int. Cl.: A61K 8/49, A61Q 5/10, A61K 8/19

(54) **OXIDATIONSFÄRBEMITTEL ZUR FÄRBUNG KERATINHALTIGER FASERN MIT LUFTSAUERSTOFF ALS EINZIGEM OXIDATIONSMITTEL**
OXIDATION DYES FOR DYEING KERATIN FIBERS CONTAINING ATMOSPHERIC OXYGEN AS THE ONLY OXIDIZING AGENT
COLORANTS D'OXYDATION POUR LA COLORATION DE FIBRES KÉRATINIQUES AVEC L'OXYGÈNE DE L'AIR COMME UNIQUE AGENT OXYDANT

(30) Priorität: 06.07.2006 DE 102006031502
(43) Veröffentlichungstag der Anmeldung: 25.03.2009
(73) Patentinhaber: Henkel AG & Co. KGaA, 40589 Düsseldorf (DE)
(72) Erfinder: SÜNGER, Georg, 40589 Düsseldorf (DE); HOLLENBERG, Detlef, 40699 Erkrath (DE); KAINZ, Sabine, 47447 Moers (DE); KOLONKO, Claudia, 42857 Remscheid (DE); EHLERT, Manuela, 51379 Leverkusen (DE)
(86) Internationale Anmeldenummer: PCT/EP2007/056657
(87) Internationale Veröffentlichungsnummer: WO 2008/003686

(56) Entgegenhaltungen:
- EP-A- 0 335 477
- EP-A- 1 442 735
- DE-A1- 19 827 000
- FR-A- 2 681 318
- FR-A- 2 769 835
- GB-A- 2 187 210
- US-A- 3 993 436
- US-A- 4 013 404
- US-A- 5 869 692
- US-A1- 2003 208 857
- US-A1- 2004 205 906

## Beschreibung

Die vorliegende Erfindung betrifft Mittel zur Färbung keratinhaltiger Fasern sowie deren Verwendung und ein entsprechendes Haarfärbeverfahren. Die Mittel enthalten mindestens einen Farbstoffvorläufer für einen naturanalogen Farbstoff ausgewählt aus der Gruppe der Indol- bzw. Indolin-Derivate, mindestens ein pH-Puffersystem sowie mindestens ein weiteres Alkalisierungsmittel und sind frei von zusätzlichen Oxidationsmitteln.

Menschliche Haare werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so dass eine vielfach unerwünschte Nuancenverschiebung oder gar eine sichtbare "Entfärbung" eintreten kann.

Eine weitere Möglichkeit keratinhaltige Fasern zu färben, bietet die Verwendung von Färbemitteln, die eine Kombination aus Komponente
A reaktive Carbonylverbindungen, d.h. Verbindungen, mit mindestens einer reaktiven Carbonylgruppe, und Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden
enthalten. Die vorgenannten Komponenten A und B sind im Allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich diese Färbemethode (im Folgenden Oxofärbung genannt) ohne weiteres mit dem oxidativen Färbesystem kombinieren. Die Komponenten A und B werden im Folgenden als Oxofarbstoffvorprodukte bezeichnet. Die Oxofärbung wird beispielweise in den Druckschriften WO-A1-99/18916, WO-A1-00/38638, WO-A1-01/34106 und WO-A1-01/47483 beschrieben.

In den Druckschriften WO-A2-99/18916 und WO-A1-01/47483 wird die Verwendung von Oniumaldehyden und -ketonen, insbesondere von 2- bzw. 4-Formyl-1-methylchinolinium-Verbindungen, zum Färben keratinhaltiger Fasern offenbart, die in Kombination mit Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aciden Verbindungen eingesetzt werden.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, vom sogenannten Entwickler-Typ (Entwicklerkomponente) und Kuppler-Typ (Kupplerkomponente). Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Schließlich hat ein weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß.

Vorzugsweise erfolgt die Ausfärbung schonend mit Luftsauerstoff. Die dabei üblicherweise verwendeten Farbstoffvorprodukte auf Indol- bzw. Indolin-Basis werden zu diesem Zweck in einen kosmetischen Träger eingearbeitet, der bevorzugt einen basischen pH-Wert aufweist. Die Ausfärbung gemäß dieser Methode resultiert in eine natürliche Schwarz-, Braun- bzw. Blondfärbung der keratinhaltigen Faser, welche jedoch insbesondere im Braun- und Blondbereich eine leichte rötliche, bläuliche oder violette Farbnuance aufweist. Finden zusätzliche Oxidationsmittel in den Färbemitteln Verwendung, treten diese Farbverschiebungen in vernachlässigbarem Maße bis gar nicht auf.

Färbemittel auf Basis der naturanalogen Farbstoffe sprechen denjenigen Verbraucher an, der auf schonendem Wege seinem ergrautem Haar eine natürliche Haarfarbe zurückverleihen möchte. Die zuvor erwähnte Farbverschiebung ist insbesondere für diesen Verbraucher unerwünscht. Daher ist die Färbung mit naturanalogen Farbstoffen in dieser Hinsicht verbesserungswürdig.

Desweiteren wurde festgestellt, dass die Färbekraft der Färbemittel mit naturanalogen Farbstoffen über eine Lagerzeit von etwa 3 Wochen und mehr abnimmt. Solche Lagerzeiten bilden bei gewerblicher Nutzung dieser Färbemittel keine Ausnahme.

Aus der Druckschrift EP-B1-1 098 627 sind Haarfärbemittel auf Basis von Farbstoffvorprodukten vom Indol- bzw. Indolin-Typ bekannt, die zur Verbesserung der Färbungen auf ergrautem Haar zusätzlich mindestens eine Aminosäure oder ein Oligopeptid enthalten. Die erzielten Färbungen besitzen auf blondem Haar allesamt einen unerwünschten Rot- oder Blaustich.

Aus der Druckschrift EP-B1-613 366 sind Haarfärbemittel bekannt, die neben einem Farbstoffvorprodukt vom Indolin-Typ, 0.05 bis 5 Gew.% mindestens eines Oxidationsfarbstoffvorprodukts vom Entwicklertyp sowie 0.05 bis 5 Gew.% mindestens eines Oxidationsfarbstoffvorprodukts vom Kupplertyp enthalten. Es wurde gefunden, dass die Indolinderivate die Färbeeigenschaften der herkömmlichen Oxidationsfärbemittel auf Entwickler- und Kupplerbasis verbessern.

Die Druckschrift EP-A2-1 254 650 offenbart Haarfärbemittel, die neben Indolinderivaten als Farbstoffvorprodukt mindestens ein ausgewähltes, organisches primäres Amin als Alkalisierungsmittel enthalten. Bei Anwendung dieser Färbemittel sollen Braun- bis Schwarzfärbungen ohne rötliche Farbverschiebung erhalten werden.

Aufgabe der vorliegenden Erfindung ist es daher, ein Färbemittel auf Basis von naturanalogen Farbstoffen bereitzustellen, welches, insbesondere auch nach längerer Lagerung, unter Luftoxidation keratinhaltigen Fasern, farbintensivere natürliche Blond-, Braun- oder Schwarztöne verleiht. Die Färbungen fallen dabei bevorzugt ohne unerwünschte rötliche, bläuliche oder violette Farbverschiebungen aus. Ferner sollten die Färbungen langlebig sein und sich rasch entwickeln.

Es wurde überraschenderweise gefunden, dass Färbemittel auf Basis von Indol- bzw. Indolinderivaten unter Luftoxidation auch nach längeren Lagerungszeiten zu intensiven Färbungen führen, wenn neben mindestens einem Alkalisierungsmittel zusätzlich mindestens ein pH-Puffersystem in diesem Mittel enthalten sind und die Mittel frei sind von zusätzlichen Oxidationsmitteln.

Ein erster Gegenstand der Erfindung ist daher ein Mittel zur durch Luftsauerstoff induzierten Färbung keratinhaltiger Fasern, insbesondere menschlicher Haare, das in einem kosmetisch akzeptablen Träger
(a) als Farbstoffvorprodukt eines naturanalogen Farbstoffs mindestens ein Indolinderivat, ausgewählt aus N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin und/oder deren physiologisch verträglichen Salzen mit anorganischen oder organischen Säuren,
(b) mindestens ein pH-Puffersystem, ausgewählt aus mindestens einem Vertreter aus der Gruppe, die gebildet wird aus Hydrogencarbonat, Carbonat, Hydrogen-carbonat/Carbonat, Citronensäure/Monohydrogenphosphat, Citronensäure/Dihydrogenphosphat, Dihydrogenphosphat und Monohydrogenphosphat/Dihydrogenphosphat, und
(c) mindestens ein weiteres Alkalisierungsmittel enthält,
mit der Maßgabe, dass kein zusätzliches Oxidationsmittel für die farbbildenden Komponenten enthalten ist.

Die Indolin Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Die Vorstufen naturanaloger Farbstoffe sind in den erfindungsgemäßen Mitteln bevorzugt in einer Menge von 0.01 bis 10 Gew.-%, insbesondere von 0.1 bis 5 Gew.-%, jeweils bezogen auf das Gewicht des anwendungsbereiten Färbemittels, enthalten.

Die erfindungsgemäßen Mittel enthalten als Komponente (b) mindestens ein pH-Puffersystem.

Als pH-Puffersystem werden erfindungsgemäß solche chemische Verbindungen bzw. eine Kombination aus chemischen Verbindungen angesehen, die In einer Lösung bewirken, dass sich der pH-Wert der Lösung bei Zugabe einer kleinen Menge Säure oder Lauge zu einem Volumen des kosmetischen Trägers nur geringfügig ändert. Diese Änderung Ist weniger ausgeprägt, als dies bei einer Zugabe der gleichen Menge an Säure oder Lauge zu einem gleichen Volumen des kosmetischen Trägers ohne pH-Puffersystem der Fall ist.

Solche pH-Puffersysteme werden ausgewählt aus mindestens einem Vertreter aus der Gruppe, die gebildet wird, aus Hydrogencarbonat, Carbonat, Hydrogencarbonat/Carbonat, Citronensäure/Monohydrogenphosphat, Citronensäure/Dihydrogenphosphat, Dihydrogenphosphat und Monohydrogenphosphat/Dihydrogenphosphat. Ganz besonders bevorzugt Ist Hydrogencarbonat/Carbonat, insbesondere in einem Molvarhältnis von 0.5 zu 1 bis 1 zu 0.5.

Die mit dem Schrägstrich gekennzeichneten pH-Puffersysteme aus obiger Liste stellen Gemische dieser durch den Schrägstrich getrennten Verbindungen dar. Die in der Liste angegebenen anionischen Verbindungen werden in Form deren Salze mit einem korrespondierenden ein- oder mehrwertigen Kation eingesetzt. Bevorzugte Kationen sind Alkalimetallkationen (insbesondere Natrium oder Kalium) und Ammoniumionen. Erfindungsgemäß verwendbare Genußsäuren sind beispielsweise Citronensäure, Weinsäure oder Äpfelsäure bzw. deren Gemische.

Das pH-Puffersystem ist bevorzugt in einer Menge von 0,1 bis 1,0 Gew.-%, besonders bevorzugt von 0,2 bis 0,8 Gew.-%, ganz besonders bevorzugt von 0,3 bis 0,7 Gew.-%, jeweils bezogen auf das Gewicht des Mittels, in dem erfindungsgemäßen Mittel enthalten.

Das gebrauchsfertige erfindungsgemäße Haarfärbepräparat sollte bevorzugt einen pH-Wert im Bereich von 6 bis 12, besonders bevorzugt von pH 8 bis pH 9,5, ganz besonders bevorzugt von pH 8,8 bis pH 9,3, aufweisen. Besonders bevorzugt ist generell die Anwendung der Haarfärbemittel in einem alkalischen Milieu, das heißt bei einem pH-Wert von größer 7.

Die erfindungsgemäß verwendbaren Alkalisierungsmittel (c) sind erfindungsgemäß von dem pH-Puffersystem (b) verschieden und werden bevorzugt ausgewählt aus der Gruppe, die gebildet wird, aus Ammoniak, basischen Aminosäuren, Alkalihydroxiden, Alkanolaminen, Alkalimetallmetasilikaten, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Zusätzlich können die erfindungsgemäßen Mittel zusätzlich Oxidationsfarbstoffvorprodukte vom Entwicklertyp umfassen, die wiederum bevorzugt in einer Menge von 0.01 bis 5 Gew.-%, insbesondere von 0.1 bis 3 Gew.-%, jeweils bezogen auf das Gewicht des anwendungsbereiten Färbemittels, enthalten sind.

Es kann erfindungsgemäß bevorzugt sein, die erfindungsgemäßen Entwicklerkomponenten aus der Gruppe auszuwählen, die gebildet wird, aus p-Phenylendiaminderivaten, zweikernigen Entwicklerkomponenten, p-Aminophenol und seinen Derivaten, Pyrimidinderivaten, Pyrazolderivaten sowie Pyrazozlopyrimidinderivaten und den physiologisch verträglichen Salzen dieser Verbindungen. Im folgenden werden erfindungsgemäß bevorzugte Entwicklerkomponenten genannt.

Besonders erfindungsgemäß bevorzugt sind p-Phenylendiaminderivate der Formel (E1) 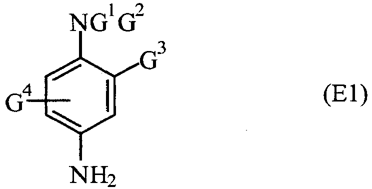 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod-oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4₋dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino-und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 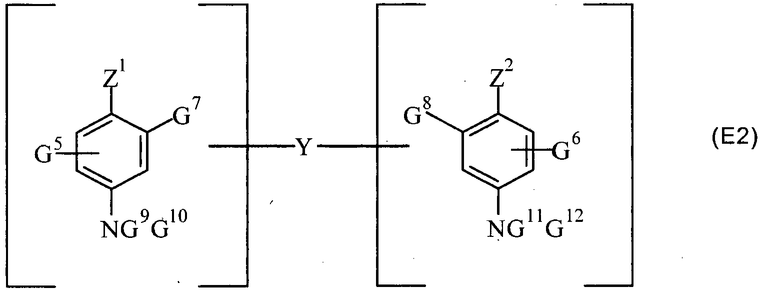 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstöffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente p-Amino-phenol oder ein p-Aminophenolderivat bzw. eines der physiologisch verträglichen Salze der vorgenannten Verbindungen einzusetzen. Besonders bevorzugt sind p-Aminophenol-derivate der Formel (E3) 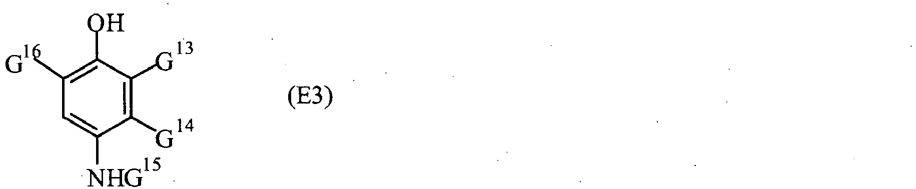 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest, einen Hydroxy-(C₁-bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Mono-hydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxy-methylamino-4-aminophenol, 4-Amino-3-hydroxymethylphenol, 4-Amino-2-(β-hydroxy-ethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazolopyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl),amino-3-amino-6-methoxy-pyridin und 3,4-Diamino-pyridin.

Bevorzugte Pyrimidin-Derivate werden erfindungsgemäß ausgewählt aus Verbindungen gemäß Formel (E4), 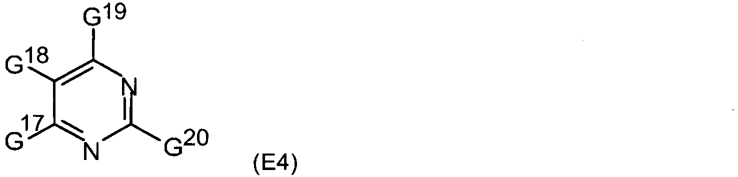 worin
- G¹⁷, G¹⁸ und G¹⁹: unabhängig voneinander für ein Wasserstoffatom, eine Hydroxygruppe, eine (C₁-C₆)-Alkoxygruppe oder eine Aminogruppe steht und
- G²⁰: für eine Hydroxygruppe oder eine Gruppe -NG²¹G²² steht, worin G²¹ und G²² unabhängig voneinander stehen für ein Wasserstoffatom, eine (C₁-C₆)-Alkylgruppe, eine (C₁-G₆)-Hydroxyalkylgruppe,
mit der Maßgabe, dass maximal zwei der Gruppen G¹⁷, G¹⁸, G¹⁹ und G²⁰ eine Hydroxygruppe bedeuten und höchstens zwei der Reste G¹⁷, G¹⁸ und G¹⁹ für ein Wasserstoffatom stehen.

Besonders bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 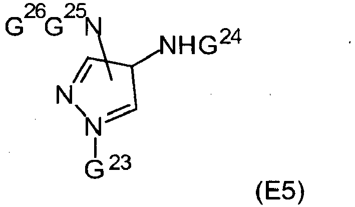 worin
- G²³, G²⁴, G²⁵: stehen unabhängig voneinander für ein Wasserstoffatom, eine C₁ bis C₆-Alkylgruppe, eine C₂ bis C₆-Monohydroxyalkylgruppe, eine C₂ bis C₆-Polyhydroxyalkylgruppe, eine gegebenenfalls substituierte Arylgruppe oder eine gegebenenfalls substituierte Aryl-C₁ bis C₆-alkylgruppe und
- G²⁶: steht für ein Wasserstoffatom, eine C₁ bis C₆-Alkylgruppe, eine C₂ bis C₆-Monohydroxyalkylgruppe oder eine C₂ bis C₆-Polyhydroxyalkylgruppe.

Besonders bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethytpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol.

Bevorzugte Pyrazolopyrimidin-Derivate sind insbesondere die Derivate des Pyrazolo[1,5-a]pyrimidins der folgenden Formel (E6) und dessen tautomeren Formen, sofern ein tautomeres Gleichgewicht besteht: 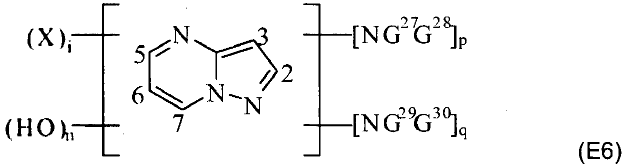 wobei:
- G²⁷, G²⁸, G²⁹ und G³⁰ unabhängig voneinander stehen für ein Wasserstoffatom, einen C₁- bis C₄-Alkylrest, einen Aryl-Rest, einen C₁- bis C₄-Hydroxyalkylrest, einen C₂-bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁-bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁-bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁-bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁-bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG²⁷G²⁸ und NG²⁹G³⁰ belegen die Positionen (2,3); (5,6); (6,7); (3,5) oder (3,7);
- wenn p + q gleich 1 ist, n den Wert 1 hat, und die Gruppen NG²⁷G²⁸ (oder NG²⁹G³⁰) und die Gruppe OH belegen die Positionen (2,3); (5,6); (6,7); (3,5) oder (3,7);

Die in Formel (E6) verwendeten Substituenten sind erfindungsgemäß analog zu den obigen Ausführungen definiert.

Wenn das Pyrazolo[1,5-a]pyrimidin der obenstehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

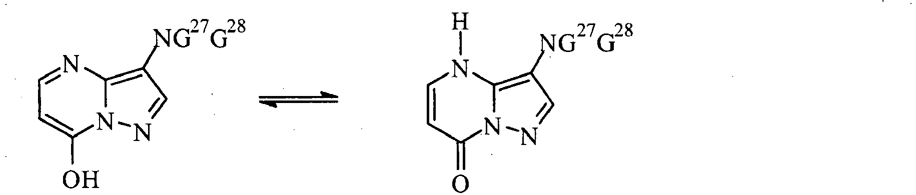

Unter den Pyrazolo[1,5-a]pyrimidinen der obenstehenden Formel (E6) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomeres Gleichgewicht vorhanden ist.

Die Pyrazolo[1,5-a]-pyrimidine der obenstehenden Formel (E6) können wie in der Literatur beschrieben durch Zyklisierung ausgehend von einem Aminopyrazol oder von Hydrazin hergestellt werden.

Erfindungsgemäß kann das erfindungsgemäße Mittel zusätzlich mindestens eine Kupplerkomponente, bevorzugt in Kombination mit mindestens einer Entwicklerkomponente, enthalten. Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5- Dihydroxynaphthalin, 2,7-Dihydroxynaphthalin und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Im Sinne der Erfindung bevorzugt geeignete Kupplerkomponenten werden ausgewählt aus der Gruppe von Verbindungen, die gebildet wird, aus
- m-Aminophenol und dessen Derivate wie bevorzugt 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Phenylendiamin und dessen Derivate wie bevorzugt 2,4-Diaminophenoxyethanol, 1,3-Bis-(2',4'-diaminophenoxy)-propan, 2-Amino-1-methoxy-4-(2-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol, 2-({3-[(2-Hydroxyethyl)amino]-2-methoxy-5-methylphenyl}amino) ethanol, 3-Amino-4-(2-methoxyethoxy)-5-methylphenylamin, 2-({3-[(2-Hydroxyethyl)amino]-4-methoxy-5-methylphenyl}amino)ethanol und 2-[(3-Morpholin-4-ylphenyl)amino]ethanol,
- o-Phenylendiamin und dessen Derivate wie bevorzugt 3,4-Diaminobenzoesäure und 2,3-Diamino-1-methylbenzol,
- Di- beziehungsweise Trihydroxybenzolderivate wie bevorzugt Resorcin und Derivate (Resorcin, Resorcinmonomethylether, 2-Methylresorcin, 5-Methylresorcin, 2,5-Dimethylresorcin, 2-Chlorresorcin, 4-Chlorresorcin), Pyrogallol und 1,2,4-Trihydroxybenzol,
- Pyridinderivate wie bevorzugt 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie bevorzugt 6-Hydroxybenzomorpholin und 6-Aminobenzomorpholin,
- Chinoxalinderivate wie bevorzugt 6-Methyl-1,2,3,4-tetrahydrochinoxalin,
- Pyrazolonderivate wie bevorzugt 1-Phenyl-3-methylpyrazol-5-on und
- Methylendioxybenzolderivate wie bevorzugt 1-Hydroxy-3,4-methylendioxybenzol, 1-Amino-3,4-methylendioxybenzol und 1-(2'-Hydroxyethyl)-amino-3,4-methylendioxybenzol.

Falls Entwickler- und Kupplerkomponenten dem erfindungsgemäßen Mittel zugesetzt werden, ist es bevorzugt, die Farbstoffvorprodukte naturanaloger Farbstoffe vom Indol- bzw. Indolintyp mit mindestens einer der folgenden Kombinationen von Entwickler und Kupplerkomponenten gemäß Tabelle 1 zu kombinieren:

**Tabelle 1: bevorzugte Kombinationen von Entwickler- und Kupplerkomponenten**

| **Kombination** | **Entwicklerkomponente** | **Kupplerkomponente** |
|---|---|---|
| K1 | p-Phenylendiaminderivat, bevorzugt gemäß Formel (E1) | Resorcin und Derivate davon |
| K2 | Pyrazolderivat, bevorzugt gemäß Formel (E5) | Resorcin und Derivate davon |
| K3 | Pyrimidinderivat, bevorzugt gemäß Formel (E4) | Resorcin und Derivate davon |
| K4 | Zweikerniger Entwickler bevorzugt gemäß Formel (E2) | Resorcin und Derivate davon |
| K5 | p-Aminophenolderivat bevorzugt gemäß Formel (E3) | m-Phenylendiamin und Derivate davon |

| | | |
|---|---|---|
| K6 | Zweikerniger Entwickler bevorzugt gemäß Formel (E2) | m-Phenylendiamin und Derivate davon |
| K7 | p-Phenylendiaminderivat bevozugt gemäß Formel (E1) | Pyridinderivat |
| K8 | Zweikerniger Enrtwickler bevorzugt gemäß Formel (E2) | Pyridinderivat |
| K9 | p-Aminophenolderivat bevorzugt gemäß Formel (E3) | Pyridinderivat |
| K10 | Zweikerniger Entwickler bevorzugt gemäß Formel (E2) | m-Aminophenol und Derivate davon |
| K11 | Pyrazolderivat, bevorzugt gemäß Formel (E5) | m-Aminophenol und Derivate davon |
| K12 | p-Phenylendiaminderivat bevorzugt gemäß Formel (E1) | 5-(2-Hydroxyethylamino)-2-methyl-phenol |

Die jeweiligen Entwickler- bzw. Kupplerkomponenten gemäß Tabelle 1 werden bevorzugt aus deren zuvor im Rahmen der jeweiligen Verbindungsklassen genannten bevorzugten individuellen Vertretern und/oder deren physiologisch verträglichen Salzen ausgewählt. Im Rahmen der speziellen Ausführungsform ist es ganz besonders bevorzugt, dass die erfindungsgemäßen Mittel mindestens eine der folgenden Entwickler/Kuppler-Kombinationen enthalten: Besonders bevorzugte Kombinationen gemäß K1 aus Tabelle 1 sind:
p-Toluylendiamin und Resorcin
2-(β-Hydroxyethyl)-p-phenylendiamin und Resorcin
p-Toluylendiamin und 2-Methylresorcin
2-(β-Hydroxyethyl)-p-phenylendiamin und 2-Methylresorcin
p-Toluylendiamin und 4-Chlorresorcin
2-(β-Hydroxyethyl)-p-phenylendiamin und 4-Chlorresorcin
2-(β-Hydroxyethyl)-p-phenylendiamin, 2-Methylresorcin und 2-Amino-3-hydroxypyridin
Besonders bevorzugte Kombinationen gemäß K2 aus Tabelle 1 sind:
4,5-Diamino-1-(2-hydroxyethyl)pyrazol und Resorcin
4,5-Diamino-1-(2-hydroxyethyl)pyrazol und 2-Methylresorcin
4,5-Diamino-1-(2-hydroxyethyl)pyrazol und 4-Chlorresorcin

Besonders bevorzugte Kombinationen gemäß K3 aus Tabelle 1 sind:
2,4,5,6-Tetraaminopyrimidin und Resorcin
2,4,5,6-Tetraaminopyrimidin und 2-Methylresorcin
2,4,5,6-Tetraaminopyrimidin und 4-Chlorresorcin
4-Hydroxy-2,5,6-triaminopyrimidin und Resorcin
4-Hydroxy-2,5,6-triaminopyrimidin und 2-Methylresorcin
4-Hydroxy-2,5,6-triaminopyrimidin und 4-Chlorresorcin

Besonders bevorzugte Kombinationen gemäß K4 aus Tabelle 1 sind:
Bis-(2-hydroxy-5-aminophenyl)-methan und Resorcin
Bis-(2-hydroxy-5-aminophenyl)-methan und 2-Methylresorcin
Bis-(2-hydroxy-5-aminophenyl)-methan und 4-Chlorresorcin

Besonders bevorzugte Kombinationen gemäß K5 aus Tabelle 1 sind:
p-Aminophenol und 2,4-Diaminophenoxyethanol
p-Aminophenol und 2-Amino-1-methoxy-4-(2-hydroxyethylamino)benzol
p-Aminophenol und 2,6-Bis-(2'-hydroxyethylamino)-1-methylbenzol
4-Amino-3-methylphenol und 2,4-Diaminophenoxyethanol
4-Amino-3-methylphenol und 2-Amino-1-methoxy-4-(2-hydroxyethylamino)benzol
4-Amino-3-methylphenol und 2,6-Bis-(2'-hydroxyethylamino)-1-methylbenzol

Besonders bevorzugte Kombinationen gemäß K6 aus Tabelle 1 sind:
Bis-(2-hydroxy-5-aminophenyl)-methan und 2,4-Diaminophenoxyethanol
Bis-(2-hydroxy-5-aminophenyl)-methan und 2-Amino-1-methoxy-4-(2-hydroxyethyl-amino)benzol
Bis-(2-hydroxy-5-aminophenyl)-methan und 2,6-Bis-(2'-hydroxyethylamino)-1-methylbenzol

Besonders bevorzugte Kombinationen gemäß K7 aus Tabelle 1 sind:
p-Toluylendiamin und 2-Amino-3-hydroxypyridin
2-(β-Hydroxyethyl)-p-phenylendiamin und 2-Amino-3-hydroxypyridin
p-Toluylendiamin und 2,6-Dihydroxy-3,4-dimethylpyridin
2-(β-Hydroxyethyl)-p-phenylendiamin und 2,6-Dihydroxy-3,4-dimethylpyridin
2-(β-Hydroxyethyl)-p-phenylendiamin, 2-Amino-3-hydroxypyridin und 2-Methylresorcin

Besonders bevorzugte Kombinationen gemäß K8 aus Tabelle 1 sind:
Bis-(2-hydroxy-5-aminophenyl)-methan und 2-Amino-3-hydroxypyridin
Bis-(2-hydroxy-5-aminophenyl)-methan und 3-Amino-2-methylamino-6-methoxypyridin
Bis-(2-hydroxy-5-aminophenyl)-methan und 2,6-Dihydroxy-3,4-dimethylpyridin
Bis-(2-hydroxy-5-aminophenyl)-methan und 3,5-Diamino-2,6-dimethoxypyridin

Besonders bevorzugte Kombinationen gemäß K9 aus Tabelle 1 sind:
p-Aminophenol und 2-Amino-3-hydroxypyridin
4-Amino-3-methylphenol und 2-Amino-3-hydroxypyridin
p-Aminophenol und 3-Amino-2-methylamino-6-methoxypyridin
4-Amino-3-methylphenol und 3-Amino-2-methylamino-6-methoxypyridin
p-Aminophenol und 2,6-Dihydroxy-3,4-dimethylpyridin
4-Amino-3-methylphenol und 2,6-Dihydroxy-3,4-dimethylpyridin
p-Aminophenol und 3,5-Diamino-2,6-dimethoxypyridin
4-Amino-3-methylphenol und 3,5-Diamino-2,6-dimethoxypyridin

Die Kombination aus
i) einem p-Phenylendiaminderivats, bevorzugt ausgewählt aus Verbindungen der Formel (E1),
ii) einem Pyridinderivat und
iii) Resorcin oder einem Derivat davon,
insbesondere die Kombination 2-(β-Hydroxyethyl)-p-phenylendiamin, 2-Amino-3-hydroxypyridin und 2-Methylresorcin, ist in den erfindungsgemäßen Mitteln ganz besonders bevorzugt enthalten.

Bei allen zuvor genannten bevorzugten Kombinationen können ebenfalls die physiologisch verträglichen Salze der entsprechend aufgezählten Verbindungen verwendet werden.

Die Oxidationsfarbstoffvorprodukte vom Kupplertyp, im folgenden auch als Kupplerkomponente bezeichnet, sind erfindungsgemäß bevorzugt in einer Menge von 0.01 bis 5 Gew.-%, insbesondere von 0.1 bis 3 Gew.-%, jeweils bezogen auf das Gewicht des anwendungsbereiten Färbemittels, enthalten.

Die Farbstoffvorprodukte naturanaloger Farbstoffe (a) und die Entwicklerkomponenten sind bevorzugt in einem Molverhältnis von 10 zu 1 bis 1 zu 2, besonders bevorzugt in einem Molverhältnis von 8 zu 1 bis 2 zu 1, ganz besonders bevorzugt in einem Molverhältnis von 6 zu 1 bis 3 zu 1 in den erfindungsgemäßen Mitteln enthalten.

Die Kupplerkomponenten und die Entwicklerkomponenten sind bevorzugt in einem Molverhältnis von 8 zu 1 bis 1 zu 2, besonders.bevorzugt in einem Molverhältnis von 6 zu 1 bis 2 zu 1, ganz besonders bevorzugt in einem Molverhältnis von 2,5 zu 1 bis 4,5 zu 1 in den erfindungsgemäßen Mitteln enthalten.

Die Farbstoffvorprodukte naturanaloger Farbstoffe (a) und die Kupplerkomponenten sind bevorzugt in einem Molverhältnis von 2 zu 1 bis 1 zu 2, besonders bevorzugt in einem Molverhältnis von 1,5 zu 1 bis 1 zu 1,5, ganz besonders bevorzugt in einem Molverhältnis von 1,5 zu 1 bis 1 zu 1 in den erfindungsgemäßen Mitteln enthalten.

Als kosmetisch akzeptabler Träger wird insbesondere ein ansonsten üblicher Träger von Mitteln zur Färbung menschlicher Haare verstanden. Die erfindungsgemäßen Färbemittel können mit Rücksicht auf die erfindungswesentlichen Merkmale entsprechend bekannter Färbemittel zusammengesetzt sein bzw. die für diese üblichen Inhaltsstoffe enthalten. Beispiele weiterer geeigneter und erfindungsgemäß bevorzugter Inhaltsstoffe sind nachstehend angegeben.

Die erfindungsgemäßen Mittel enthalten die erfindungsgemäßen Komponenten bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Eine allgemeine Zusammensetzung ist nachstehend angegeben, mit der Maßgabe, dass das erfindungsgemäße Stoffmengenverhältnis von Verbindung A zu Verbindung B eingehalten wird:
Neben den erfindungsgemäßen Verbindungen können die erfindungsgemäßen Färbemittel in einer weiteren Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

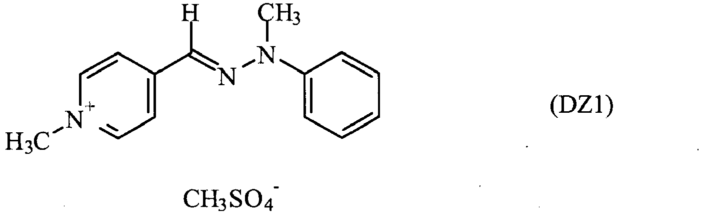

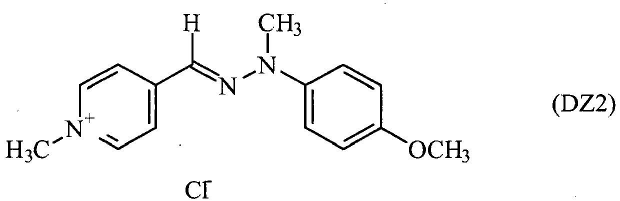

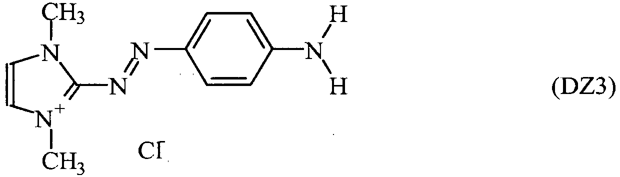

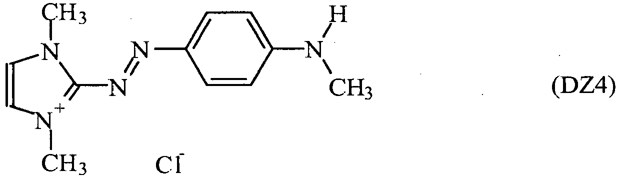

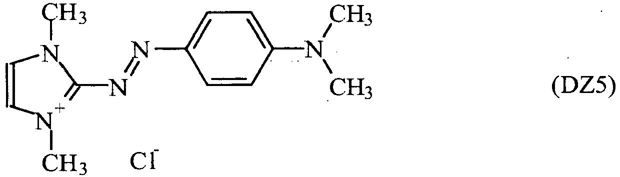

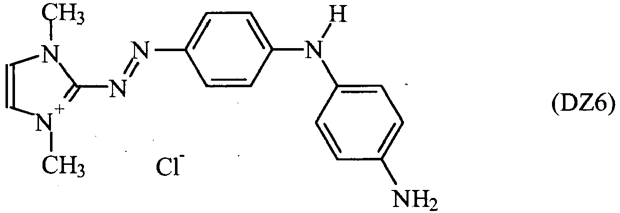

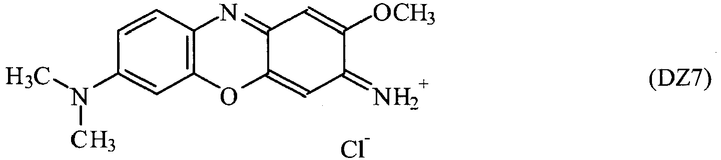

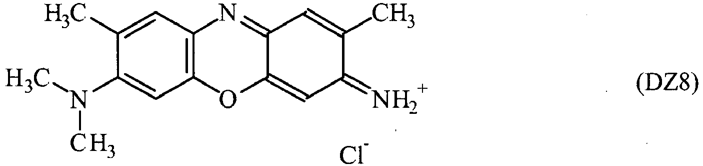

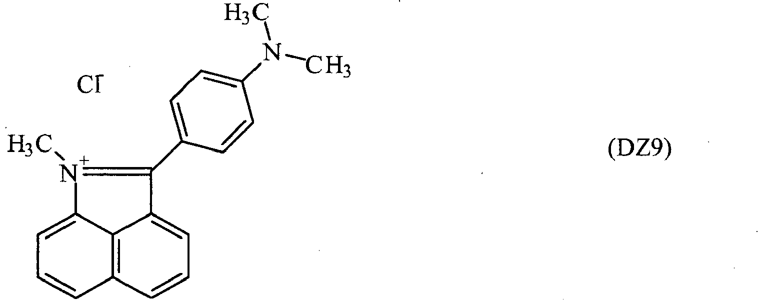

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter der Marke Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Verbindungen A bzw. 2 oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, -phosphaten und/oder -isethionaten, die sich von Alkyl- und/oder Alkenyloligoglykosiden der allgemeinen Formel (II) ableiten,

   R-O-(G)ₚ (II)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10,
   insbesondere das Laurylglucosidcarboxylat, wie es als Plantapon^{®} LGC von Cognis Deutschland erhältlich ist,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder-SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß können als kationische Tenside insbesondere solche vom Typ der quartären Ammoniumverbindungen, der Esterquats und der Amidoamine eingesetzt werden.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Marken Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen; vom jeweiligen Rohstoff abhängigen Alkylkettenlängen erhält.

Bei den Tensiden, die Anlagerungsprodukte von Ethylen-und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

In einer weiteren bevorzugten Ausführungsform enthalten die erfindungsgemäßen Mittel zusätzlich mindestens ein kationisches und oder mindestens ein amphoteres Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₄-Kohlenwasserstoffgruppe an eine aus Acrylsäure, Methacrylsäure oder deren Derivaten aufgebaute Polymerhauptkette gebunden sind, haben sich als besonders geeignet erwiesen.
Homopolymere der allgemeinen Formel (III), 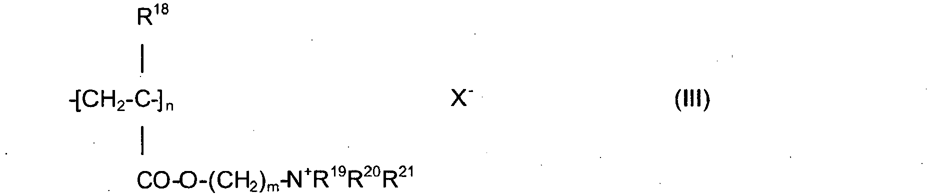 in der R¹⁸ = -H oder-CH₃ ist, R¹⁹, R²⁰ und R²¹ unabhängig voneinander ausgewählt sind aus C₁₋₄-Alkyl-, -Alkenyl- oder-Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxy-propylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1 Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50,
- kationische Guar-Derivate, wie insbesondere die unter den Handelsnamen Cos-media^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie. Abil^{®}-Quat 3270 und 3272 (Hersteller: Th. Goldschmidt; diquaternäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merrtuat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylamino-alkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
sowie die unter den Bezeichnungen
- Polyquaternium 2 (z.B. Mirapol^{®} A-15 der Firma Rhodia),
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Unter dem Begriff amphotere Polymere werden solche Polymere verstanden,
- die im Molekül sowohl freie Aminogruppen als auch freie -COOH- oder SO₃H-Gruppen enthalten und zur Ausbildung innerer Salze befähigt sind,
- zwitterionische Polymere, die im Molekül quartäre Ammoniumgruppen und -COO⁻-Gruppen oder -SO₃-Gruppen enthalten, sowie
- Polymere, die -COOH-Gruppen oder SO₃H-Gruppen und quartäre Ammoniumgruppen enthalten.
Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere und/oder kationische Polymere sind solche Polymerisate, in denen sich eine kationische Gruppe ableitet von mindestens einem der folgenden Monomere:
(M1) Monomeren mit quartären Ammoniumgruppen der allgemeinen Formel (IV),

   R¹-CH=CR²-CO-Z-(CₙH₂ₙ)-N⁽⁺⁾R³R⁴R⁵ A⁽⁻⁾ (IV)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist,
(M2) Monomeren mit quartären Ammoniumgruppen der allgemeinen Formel (V), 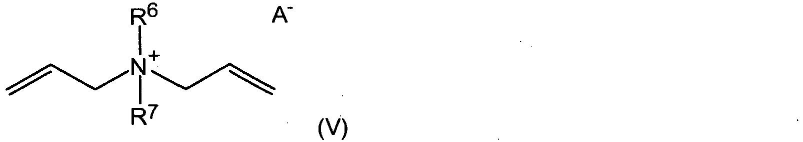
   worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist.

Wenn sich eine kationische Gruppe der amphoteren bzw. kationischen Polymerisate vom Monomer des Typs (M1) ableitet, stehen in Formel (IV) die Reste R³, R⁴ und R⁵ bevorzugt für Methylgruppen, Z ist bevorzugt eine NH-Gruppe und A⁽⁻⁾ bedeutet bevorzugt ein Halogenid-, Methoxysulfat- oder Ethoxysulfat-Ion. Besonders bevorzugt ist es in diesem Falle Acrylamidopropyl-trimethyl-ammoniumchlorid als Monomer (M1) zu verwenden.

In Formel (V) steht A- bevorzugt für ein Halogenidion, insbesondere für Chlorid oder Bromid.

Bevorzugte erfindungsgemäße amphotere Polymere sind Polymere, deren anionische Gruppe sich von mindestens einem Monomeren (M3) ableitet
(M3) monomeren Carbonsäuren der allgemeinen Formel (VI) bzw. deren Salze mit einer organischen oder anorganischen Säure,

   R⁸-CH=CR⁹-COOH (VI)

   in denen R⁸ und R⁹ unabhängig voneinander Wasserstoff oder Methylgruppen sind.

Als Monomeres (M3) wird für die erfindungsgemäß bevorzugten amphoteren Polymerisate Acrylsäure verwendet.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben.

Darüber hinaus können die erfindungsgemäßen amphoteren Polymere neben einem Monomer (M1) oder M(2) und einem Monomer (M3) zusätzlich ein Monomer (M4)
(M4) monomere Carbonsäureamide der allgemeinen Formel (VII), 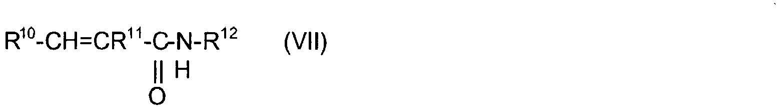 in denen R¹⁰ und R¹¹ unabhängig voneinander Wasserstoff oder Methylgruppen sind und R¹² für ein Wasserstoffatom oder eine (C₁- bis C₈)-Alkylgruppe steht, enthalten.

Erfindungsgemäß ganz besonders bevorzugt verwendete amphotere Polymere auf Basis eines Comonomers (M4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Besonders bevorzugt eingesetzte amphotere Polymere sind solche Polymerisate, die sich im wesentlichen zusammensetzen aus
(i) Monomeren mit quartären Ammoniumgruppen der allgemeinen Formel (IV),

   R¹-CH=CR²-CO-Z-(CₙH₂ₙ)-N⁽⁺⁾R²R³R⁴ A⁽⁻⁾ (IV)

   in der R¹ und R² unabhängig voneinander stehen für Wasserstoff oder eine Methylgruppe und R³, R⁴und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(ii) monomeren Carbonsäuren der allgemeinen Formel (VI),

   R⁸-CH=CR⁹-COOH (VI)

   in denen R⁰ und R⁹ unabhängig voneinander Wasserstoff oder Methylgruppen sind.

Bezüglich der Einzelheiten der Herstellung dieser besonders bevorzugten Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (i) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethy-ammoniumchlorid ist ein besonders bevorzugtes Monomeres (i). Als Monomeres (ii) für die genannten Polymerisate wird bevorzugt Acrylsäure verwendet.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinyl-ether/Malein-säureanhydrid-Copolymere und Acrylsäure/Ethylacrylat/N-tert.Butylacrylamid-Terpolymere,
- amphiphile Polymere, wie beispielsweise die Polymere gemäß der INCI-Bezeichnung:Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß kann aber das Färbemittel auch zusammen mit einem Oxidationsaktivator auf das Haar aufgebracht werden, der die Oxidation der Farbstoffvorprodukte durch den Luftsauerstoff aktiviert. Die Oxidationsaktivatoren sind bevorzugt ausgewählt aus der Gruppe, die gebildet wird aus Carbonaten, Hydrogencarbonaten, Carbamaten, Carbonsäureestern oder deren Salze, Aldehyden, insbesondere aliphatischen Aldehyden, 1,3-Dihydroxyaceton, Imidazol und seinen Derivaten, Alkali- und Ammoniumperoxidisulfaten, Metallionen, Iodiden, Chinonen und Enzymen.

Da die oxidative Färbung durch Luftsauerstoff ausgebildet wird, kann es erfindungsgemäß vorteilhaft sein, als Oxidationsaktivator Metallionen zu verwenden.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Die Aktivatoren sind bevorzugt in Mengen von 0.01 bis 5 Gew.%, bezogen auf das Gewicht des gesamten Färbemittels, in den erfindungsgemäßen Mitteln enthalten.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger kosmetischer Träger, z.B. ein Färbeshampoo, verwendet wurde.

Ein zweiter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Die Einwirkzeit beträgt bevorzugt 1 bis 30 Minuten, besonders bevorzugt 1 bis 15 Minuten, ganz besonders bevorzugt 2 bis 10 Minuten.

Zur graduellen Anpassung von ergrautem Haar an die ursprüngliche Naturhaarfarbe eines Probanden ist es erfindungsgemäß bevorzugt, das ergraute Haar in Abständen von einem bis mehreren Tagen, insbesondere 1 bis 14 Tagen, wiederholt wie oben beschrieben mit dem erfindungsgemäßen Mittel des ersten Erfindungsgegenstandes zu behandeln. Bei dieser kontinuierlichen Wiederholung des erfindungsgemäßen Verfahrens sollten allerdings geringere Einwirkzeiten von bevorzugt 1 bis 15 Minuten, besonders bevorzugt 2 bis 10 Minuten, ganz besonders bevorzugt 3 bis 5 Minuten, eingehalten werden.

Bei Probanden mit teilweise ergrautem Haar kann es erfindungsgemäß bevorzugt sein, nur die ergrauten Haarpartien mit dem erfindungsgemäßen Mittel des ersten Erfindungsgegenstandes zu behandeln. Zu diesem Zweck bedient sich der Proband selbst oder ein Friseur einer Applikationshilfe, bevorzugt in Form eines kleinen Pinsels oder einer Mascarabürste. Hier ist es erfindungsgemäß bevorzugt, die oben beschriebene wiederholte Anwendung zur graduellen Farbanpassung zu wählen.

Das erfindungsgemäße Haarfärbemittel wird zu gewerblichen Zwecken in einer Verkaufseinheit angeboten. Diese Verkaufseinheit (Kit) enthält mindestens einen Container, der das erfindungsgemäße Mittel des ersten Erfindungsgegenstands enthält. Wenn der Verbraucher das Verfahren zur graduellen Anpassung der Haarfarbe wählen soll, so kann es erfindungsgemäß bevorzugt sein, dass das Kit für jede beabsichtigte Anwendung einen separaten Container enthaltend das erfindungsgemäße Mittel der ersten Erfindungsgegenstandes, enthält. Folglich würde das Kit in letztgenannter Ausführungsform mehr als einen Container mit dem erfindungsgemäßen Mittel enthalten.

Das Kit kann zusätzlich Applikationshilfen enthalten, insbesondere die zuvor beschriebenen Applikationshilfen.

Das Kit kann zusätzlich Schutzhandschuhe enthalten.

Das Kit kann weiterhin zusätzlich einen Konditioner und/oder ein Shampoo enthalten.

### Beispiele

Es wurden folgende erfindungsgemäße Färbemittelrezepturen E1 bis E5 und die Vergleichsrezeptur V1 (siehe Tabelle 1) hergestellt. Dabei fanden folgende Rohstoffe Verwendung:
- Hydrenol^{®} D: C₁₆-C₁₈-Fettalkohol (INCI-Bezeichnung: Cetearyl alcohol) (Cognis Deutschland)
- Lorol^{®} techn.: C₁₂-C₁₈-Fettalkohol (INCI-Bezeichnung: Coconut alcohol) (Cognis Deutschland)
- Eumulgin^{®} B1: Cetylstearylakohol mit 12 EO-Einheiten (INCI-Bezeichnung: Ceteareth-12) (Cognis Deutschland)
- Eumulgin^{®} B2: Cetylstearylalkohol mit ca. 20 EO-Einheiten (INCI-Bezeichnung: Ceteareth-20) (Cognis Deutschland)
- Akypo^{®} Soft 45 NV: C₁₂₋₁₄-Fettalkohol Essigsäure Natrium Salz mit 4.5 Einheiten Ethylenoxid (INCI-Bezeichnung: Sodium Laureth-5 Carboxylate), Aktivsubstanz 21%, (KAO)
- Texapon^{®} K 14 S: Laurylmyristylethersulfat Natrium Salz mit 3 Einheiten Ethylenoxid (INCI-Bezeichnung: Sodium Myreth Sulfate), Aktivsubstanz 70%, (Cognis Deutschland)
- Plantacare^{®} 1200 UP: C₁₂₋₁₆ Alkylglucosid (INCI-Bezeichnung: Lauryl Glucoside), 51 % Aktivsubstanz, (Cognis Deutschland)
- Eutanol^{®} G: 2-Octyldodecanol (INCI-Bezeichnung: Octyldodecanol) (Cognis Deutschland)
- AMP^{®} 95: 2-Amino-2-methylpropan-1-ol (INCI-Bezeichnung: Aminomethyl Propanol) (DOW)
- Merquat^{®} 280: Dimethyldiallylammoniumchlorid/Acrylsäure Copolymer (ca. 35 Aktivsubstanz in Wasser; INCI-Bezeichnung: Polyquaternium-22) (Nalco)
- Merquat^{®} 550: Dimethyldiallylammoniumchlorid/Acrylamid Copolymer (ca. 8.1-9.1% Aktivsubstanz in Wasser; INCI-Bezeichnung: Polyquaternium-7) (Nalco)
- Merquat^{®} 3330 Plus: Dimethyldiallylammoniumchlorid/Acrylsäure/Acrylamid Terpolymer (ca. 9,5% Festkörper in Wasser; INCI-Bezeichnung: Polyquatemium-39) (Nalco)
- Luviquat^{®} FC 550: 3-Methyl-1-vinylimidazoliumchlorid/Vinylpyrrolidon-Copolymerisat (50:50) (38-42%Festkörper in Wasser; INCI-Bezeichnung: Polyquatemium-16) (BASF)
- Polymer W 37194: ca. 20Gew.-% Aktivsubstanzgehalt in Wasser; INCI-Bezeichnung: Acrylamidopropyltrimonium Chloride/Acrylates Copolymer (Stockhausen)
- Texapon^{®} NSO UP: Laurylethersulfat, Natriumsalz (ca. 27,5% Aktivsubstanz; INCI-Bezeichnung: Sodium Laureth Sulfate) (Cognis)
- Dehyton^{®} K: N,N-Dimethyl-N-(C8-18-kokosamidopropyl)ammonium-acetobetain (ca. 30% Aktivsubstanz; INCI-Bezeichnung: Aqua (Water), Cocamidopropyl Betaine) (Cognis)
- Edenor^{®} C14: Myristinsäure (INCI-Bezeichnung: MYRISTIC ACID) (Cognis)
- Plantapon^{®} LGC sorb: ca. 28-34% Aktivsubstanzgehalt in Wasser; INCI-Bezeichnung: Lauryl Glucose Carboxylate, Lauryl Glucoside (Cognis)
- Timiron^{®} Bronze MP-60: Effektpigmentprodukt enthaltend 59-69 % Glimmer, 31-39 % Fe₂O₃ und 2-3% TiO₂; Mittlere Teilchendichte 22-37 µm (INCI-Bezeichnung: Mica, CI 77491 (Iron Oxides), CI 77891 (Titanium Dioxide)) (Merck)

**Tabelle 1: Haarfärbemittel**

| Rohstoffe | E1 Gew.-% | E2 Gew.-% | E3 Gew.-% | E4 Gew.-% | E5 Gew.-% | V1 Gew.-% |
|---|---|---|---|---|---|---|
| Hydrenol^{®} D | 8,0 | 8,0 | 8,0 | 8,0 | 8,0 | 8,0 |
| Lorol^{®} techn. | 2,4 | 2,4 | 2,4 | 2,4 | 2,4 | 2,4 |
| Eumulgin^{®} B1 | 0,5 | 0,5 | 0,5 | 0,5 | 0,5 | 0,5 |
| Eumulgin^{®} B 2 | 0,5 | 0,5 | 0,5 | 0,5 | 0,5 | 0,5 |
| Akypo^{®} Soft 45 NV | 10,0 | 10,0 | 10,0 | 10,0 | 10,0 | 10,0 |
| Texapon^{®} K 14 S | 2,8 | 2,8 | 2,8 | 2,8 | 2,8 | 2,8 |
| Plantacare^{®} 1200 UP | 2,0 | 2,0 | 2,0 | 2,0 | 2,0 | 2,0 |
| Eutanol^{®} G | 1,0 | 1,0 | 1,0 | 1,0 | 1,0 | 1,0 |
| Methylparaben | 0,2 | 0,2 | 0,2 | 0,2 | 0,2 | 0,2 |
| Propylparaben | 0,2 | 0,2 | 0,2 | 0,2 | 0,2 | 0,2 |
| L-Arginin | 0,3 | 0,3 | 0,3 | 0,3 | 0,3 | 0,3 |
| Dinatriumcarbonat | 0,5 | 0,5 | - | 0,5 | 0,5 | - |
| Natriumhydrogencarbonat | 0,5 | - | 0,5 | 0,5 | 0,5 | - |
| Polymer W 37194 | 1,0 | - | - | - | - | 1,0 |
| Merquat^{®} 280 | - | 1,0 | - | - | - | - |
| Merquat^{®} 550 | - | - | 1,0 | - | - | - |
| Merquat^{®} 3330 Plus | - | - | - | 1,0 | - | - |
| Luviquat^{®} FC 550 | - | - | - | - | 1,0 | - |
| Ascorbinsäure | 0,2 | 0,2 | 0,2 | 0,2 | 0,2 | 0,2 |
| 5,6-Dihydroxyindolin HBr | 0,8 | 0,8 | 0,8 | 0,8 | 0,8 | 0,8 |
| Ammoniak (25%ige wässrige Lösung) | 0,5 | 0,5 | 0,5 | 0,5 | 0,5 | 0,5 |
| Parfum | 0,3 | 0,3 | 0,3 | 0,3 | 0,3 | 0,3 |
| Wasser | ad 100 | ad 100 | ad 100 | ad 100 | ad 100 : | ad 100 |

Die Haarfärbemittel wurden 4 Wochen unter Luftausschluß gelagert.

Pro Haarfärbemittel gemäß Tabelle 1 wurde nach der Lagerung jeweils eine Strähne (zu 90% ergrautes Humanhaar) mit dem besagten Mittel über einem Zeitraum von 5 Minuten inkubiert (4 g Haarfärbemittel pro 1 g Haar). Die Strähnen wurden mit einer identischen Menge Wasser gespült und an der Luft getrocknet.

Dieser Färbeschritt wurde in einem Abstand von jeweils etwa 15 Stunden zweimal wiederholt.

Mit dem bloßen Auge war zu erkennen, dass die erfindungsgemäßen Färbungen E1 bis E5 allesamt eine intensivere Färbung aufwiesen, als die Färbung der Strähne, die mit der Rezeptur V1 erhalten wurde.

Folgende Rezepturen stehen ebenso exemplarisch für die Erfindung und eignen sich zu deren Durchführung:

| Rohstoffe | E6 [Gew.-%] | E7 [Gew.-%] | E8 [Gew.-%] | E9 [Gew.-%] |
|---|---|---|---|---|
| Hydreno^{®} D | 6,67 | 9,0 | 9,0 | - |
| Cetearylalkohol | - | - | - | 7,5 |
| Lorol^{®} techn. | 2,4 | 3,0 | 3,0 | 2,5 |
| Eutanol^{®} G | 1,0 | - | - | - |
| Eumulgin^{®} B1 | 0,6 | 0,5 | 0,5 | - |
| Eumulgin^{®} B 2 | 0,6 | 0,5 | 0,5 | 2,0 |
| Texapon^{®} NSO UP | 20,0 | 7,0 | 7,0 | - |
| Akypo^{®} Soft 45 NV | 10,0 | - | - | - |
| Plantacare^{®} 1200 UP | 2,0 | - | - | - |
| Dehyton^{®} K | - | - | 5,0 | - |
| Edenor^{®} C14 | - | 0,5 | 0,5 | - |
| Kaliumhydroxid | - | 1,2 | 1,2 | - |
| Isostearinsäure | - | 2,0 | 2,0 | - |
| Plantapon^{®} LGC sorb | - | 5,0 | - | - |
| 2-Phenoxyethanol | 0,5 | 0,5 | 0,5 | 0,5 |
| Methylparaben | 0,2 | 0,2 | 0,2 | 0,2 |
| Propylparaben | 0,2 | 0,2 | 0,2 | 0,2 |
| L-Arginin | 0,1 | 0,3 | 0,3 | 0,3 |
| Dinatriumcarbonat | 0,5 | 0,5 | 0,5 | 0,5 |
| Natriumhydrogencarbonat | 0,5 | 0,5 | 0,5 | 0,5 |
| Polymer W 37194 | 1,0 | 1,0 | 1,0 | 1,0 |
| Ascorbinsäure | 0,2 | 0,2 | 0,2 | 0,2 |
| 5,6-Dihydroxyindolin HBr | 1,0 | 1,0 | 1,0 | 1,0 |
| Ammoniak (25%ige wässrige Lösung) | 0,65 | 0,65 | 0,65 | 0,65 |
| Timiron^{®} Bronze MP-60 | 1,0 | 1,0 | 1,0 | 1,0 |
| Parfum | 0,3 | 0,3 | 0,3 | 0,3 |
| Wasser | ad 100 | ad 100 | ad 100 | ad 100 |

## Patentansprüche

1. Mittel zur durch Luftsauerstoff induzierten Färbung keratinhaltiger Fasern, insbesondere menschlicher Haare, das in einem kosmetisch akzeptablen Träger
(a) als Farbstoffvorprodukt eines naturanalogen Farbstoffs mindestens ein Indolinderivat, ausgewählt aus N-Methyl-5,6-dihydroxyindolin, N-Ethyl-5,6-dihydroxy-Indolln, N-Propyl-5,6-dihydroxyindolin, N-Butyl-5,6-dihydroxyindolin, 5,6-Dihydroxyindolin und/oder deren physiologisch verträglichen Salzen mit anorganischen oder organischen Sauren,
(b) mindestens ein pH-Puffersystem, ausgewählt aus mindestens einem Vertreter aus der Gruppe, die gebildet wird aus Hydrogencarbonat, Carbonat, Hydrogencarbonat/Carbonat, Citronensäure/Monohydrogenphosphat, Citronensäure/Dihydrogenphosphat, Dihydrogenphosphat und Monohydrogenphosphat/Dihydrogenphosphat, und
(c) mindestens ein weiteres Alkalisierungsmittel enthält,
mit der Maßgabe, dass kein zusätzliches Oxidationsmittel für die farbbildenden Komponenten enthalten ist.

2. Mittel nach Anspruch 1, **dadurch gekennzeichnet, dass** die Farbstoffvorprodukte des naturanalogen Farbstoffs (a) in einer Menge von 0.01 bis 10 Gew.-% bezogen auf das Gewicht des anwendungsbereiten Mittels enthalten sind.

3. Mittel nach einem der Ansprüche 1 oder 2, **dadurch gekennzeichnet, dass** das pH-Puffersystem in einer Menge von 0,1 bis 1,0 Gew.-%, besonders bevorzugt von 0,2 bis 0,8 Gew.%, ganz besonders bevorzugt von 0,3 bis 0,7 Gew.%, jeweils bezogen auf das Gewicht des Mittels, enthalten ist.

4. Mittel nach einem der Ansprüche 1 bis 3, **dadurch gekennzeichnet, dass** das Alkalisierungsmittel (c) aus der Gruppe ausgewählt wird, die gebildet wird, aus Ammoniak, basischen Aminosäuren, Alkalihydroxiden, Alkanolaminen, Alkalimetallmetasilikaten, Alkaliphosphaten und Alkalihydrogenphosphaten.

5. Mittel nach einem der Ansprüche 1 bis 4, **dadurch gekennzeichnet, dass** es einen pH-Wert im Bereich von pH 6 bis 12 besitzt.

6. Mittel nach einem der Ansprüche 1 bis 5, **dadurch gekennzeichnet, dass** zusätzlich mindestens ein Oxidationsfarbstoffvorprodukt vom Entwicklertyp enthalten ist.

7. Mittel nach einem der Ansprüche 1 bis 6, **dadurch gekennzeichnet, dass** zusätzlich mindestens ein Oxidationsfarbstoffvorprodukt vom Kupplertyp enthalten ist.

8. Mittel nach einem der Ansprüche 6 bis 7, **dadurch gekennzeichnet, dass** die Farbstoffvorprodukte naturanaloger Farbstoffe (a) und die Entwicklerkomponenten in einem Molverhältnis von 10 zu 1 bis 1 zu 2, besonders bevorzugt in einem Molverhältnis von 8 zu 1 bis 2 zu 1, ganz besonders bevorzugt in einem Molverhältnis von 6 zu 1 bis 3 zu 1 enthalten sind.

9. Mittel nach einem der Ansprüche 7 bis 8, **dadurch gekennzeichnet, dass** die die Kupplerkomponenten und Entwicklerkomponenten in einem Molverhältnis von 8 zu 1 bis 1 zu 2, besonders bevorzugt In einem Molverhältnis von 6 zu 1 bis 2 zu 1, ganz besonders bevorzugt in einem Molverhältnis von 2,5 zu 1 bis 4,5 zu 1 enthalten sind.

10. Mittel nach einem der Ansprüche 7 bis 9, **dadurch gekennzeichnet, dass** die Farbstoffvorprodukte naturanaloger Farbstoffe (a) und die Kupplerkomponenten in einem Molverhältnis von 2 zu 1 bis 1 zu 2, besonders bevorzugt in einem Molverhältnis von 1,5 zu 1 bis 1 zu 1,5, ganz besonders bevorzugt in einem Molverhältnis von 1,5 zu 1 bis 1 zu 1 enthalten sind.

11. Mittel nach einem der Ansprüche 1 bis 10, **dadurch gekennzeichnet, dass** es zusätzlich mindestens ein Tensid enthält, ausgewählt aus der Gruppe, die gebildet wird aus anionischen Tensiden, zwitterionischen Tensiden, ampholytischen Tensiden, nichtionischen Tensiden und kationischen Tensiden.

12. Verfahren zur Färbung keratinischer Fasern, bei dem ein Mittel nach einem der Ansprüche 1 bis 11 auf die Fasern aufgetragen wird und nach einer Einwirkzeit wieder abgespült wird.

## Claims

1. An agent for the atmospheric oxygen-induced coloring of keratin-containing fibers, especially human hair, which contains in a cosmetically acceptable carrier
(a) as a dye precursor of a natural-analog dye at least one indoline derivative, selected from N-methyl-5,6-dihydroxyindoline, N-ethyl-5,6-dihydroxyindoline, N-propyl-5,6-dihydroxyindoline, N-butyl-5,6-dihydroxyindoline, 5,6-dihydroxyindoline, and/or the physiologically acceptable salts with inorganic or organic acids,
(b) at least one pH buffer system selected from at least one representative from the group formed by hydrogen carbonate, carbonate, hydrogen carbonate/carbonate, citric acid/monohydrogen phosphate, citric acid/dihydrogen phosphate, dihydrogen phosphate, and monohydrogen phosphate/dihydrogen phosphate, and
(c) at least one other alkalinizing agent,
with the proviso that no additional oxidizing agent for the color-forming components is present.

2. The agent according to claim 1, **characterized in that** the dye precursors of the natural-analog dye (a) are present in an amount of 0.01 to 10% by weight, based on the weight of the ready-to-use agent.

3. The agent according to one of claims 1 or 2, **characterized in that** the pH buffer system is present in an amount of 0.1 to 1.0% by weight, especially preferably of 0.2 to 0.8% by weight, very especially preferably of 0.3 to 0.7% by weight, based in each case on the weight of the agent.

4. The agent according to one of claims 1 to 3, **characterized in that** the alkalinizing agent (c) is selected from the group, formed by ammonia, basic amino acids, alkali hydroxides, alkanolamines, alkali metal metasilicates, alkali phosphates, and alkali hydrogen phosphates.

5. The agent according to one of claims 1 to 4, **characterized in that** it has a pH in the range of pH 6 to 12.

6. The agent according to one of claims 1 to 5, **characterized in that** in addition at least one oxidation dye precursor of the developer type is present.

7. The agent according to one of claims 1 to 6, **characterized in that** in addition at least one oxidation dye precursor of the coupler type is present.

8. The agent according to one of claims 6 to 7, **characterized in that** the dye precursors of natural-analog dyes (a) and the developer components are present in a molar ratio of 10 to 1 to 1 to 2, especially preferably in a molar ratio of 8 to 1 to 2 to 1, and very especially preferably in a molar ratio of 6 to 1 to 3 to 1.

9. The agent according to one of claims 7 to 8, **characterized in that** the coupler components and developer components are present in a molar ratio of 8 to 1 to 1 to 2, especially in a molar ratio of 6 to 1 to 2 to 1, and very especially preferably in a molar ratio of 2.5 to 1 to 4.5 to 1.

10. The agent according to one of claims 7 to 9, **characterized in that** the dye precursors of natural-analog dyes (a) and the coupler components are present in a molar ratio of 2 to 1 to 1 to 2, especially preferably in a molar ratio of 1.5 to 1 to 1 to 1.5, and very especially preferably in a molar ratio of 1.5 to 1 to 1 to 1.

11. The agent according to one of claims 1 to 10, **characterized in that** it contains in addition at least one surfactant, selected from the group formed by anionic surfactants, zwitterionic surfactants, ampholytic surfactants, nonionic surfactants, and cationic surfactants.

12. A method for coloring keratinic fibers in which an agent according to one of claims 1 to 11 is applied to the fibers and rinsed off again after a treatment time.

## Revendications

1. Agent de coloration, induite par l'oxygène de l'air, de fibres de kératine, en particulier de cheveux humains, qui contient dans un support cosmétiquement acceptable,
(a) comme précurseur d'un colorant analogue aux colorants naturels, au moins un dérivé de l'indoline choisi parmi la N-méthyl-5,6-dihydroxyindoline, la N-éthyl-5,6-dihydroxyindoline, la N-propyl-5,6-dihydroxyindoline, la N-butyl-5,6-dihydroxyindoline, la 5,6-dihydroxyindoline, et/ou de leurs sels physiologiquement acceptables avec des acides minéraux ou organiques,
(b) au moins un système tampon de pH, choisi parmi au moins un représentant du groupe qui est constitué du bicarbonate, du carbonate, du bicarbonate/carbonate, de l'acide citrique/monohydrogénophosphate, l'acide citrique/dihydrogénophosphate, le dihydrogénophosphate et le monohydrogénophosphate/dihydrodihydrogénophosphate, et
(c) au moins un autre agent alcalinisant,
avec la condition qu'aucun oxydant supplémentaire pour les composants de coloration n'est contenu.

2. Agent selon la revendication 1, **caractérisé en ce que** les précurseurs du colorant analogue aux colorants naturels sont contenus (a) dans une quantité allant de 0,01 à 10% en poids par rapport au poids de l'agent prêt à l'emploi.

3. Agent selon l'une des revendications 1 ou 2, **caractérisé en ce que** le système tampon de pH est contenu dans une quantité allant de 0,1 à 1,0% en poids, de façon particulièrement préférée de 0,2 à 0,8% en poids, de façon tout particulièrement préférée de 0,3 à 0,7% en poids, à chaque fois par rapport au poids de l'agent.

4. Agent selon l'une des revendications 1 à 3, **caractérisé en ce que** l'agent alcalinisant (c) est choisi dans le groupe constitué par l'ammoniac, les acides aminés basiques, les hydroxydes alcalins, les alcanolamines, les silicates de métaux alcalins, les phosphates alcalins et les hydrogénophosphates alcalins.

5. Agent selon l'une des revendications 1 à 4, **caractérisé en ce qu'**il présente une valeur de pH dans la gamme de pH allant de 6 à 12.

6. Agent selon l'une des revendications 1 à 5, **caractérisé en ce qu'**il contient en outre au moins un précurseur de colorant d'oxydation du type développeur.

7. Agent selon l'une des revendications 1 à 6, **caractérisé en ce qu'**il contient en outre au moins un précurseur de colorant d'oxydation du type copulateur.

8. Agent selon l'une des revendications 6 à 7, **caractérisé en ce que** les précurseurs des colorants analogues aux colorants naturels (a) et les composants développeurs sont contenus dans un rapport molaire allant de 10:1 à 1:2, de manière particulièrement préférée dans un rapport molaire allant de 8:1 à 2:1, et de manière tout particulièrement préférée dans un rapport molaire allant de 6:1 à 3:1.

9. Agent selon l'une des revendications 7 à 8, **caractérisé en ce que** les composants copulateurs et les composants développeurs sont contenus dans un rapport molaire allant de 8:1 à 1:2, de manière particulièrement préférée dans un rapport molaire allant de 6:1 à 2:1, de manière tout particulièrement préférée dans un rapport molaire allant de 2,5:1 à 4,5:1.

10. Agent selon l'une des revendications 7 à 9, **caractérisé en ce que** les précurseurs de colorants analogues aux colorants naturels (a) et les composants copulateurs sont contenus dans un rapport molaire allant de 2:1 à 1:2, de manière particulièrement préférée dans un rapport molaire allant de 1,5:1 à 1:1,5, de manière tout particulièrement préférée dans un rapport molaire allant de 1,5:1 à 1:1.

11. Agent selon l'une des revendications 1 à 10, **caractérisé en ce qu'**il contient en outre au moins un agent tensioactif choisi dans le groupe constitué par les tensioactifs anioniques, les tensioactifs zwittérioniques, les tensioactifs ampholytiques, les tensioactifs non ioniques et les tensioactifs cationiques.

12. Procédé de coloration de fibres de kératine, dans lequel un agent selon l'une des revendications 1 à 11 est appliqué sur les fibres et est à nouveau rincé après un temps d'action.
